(19) Europäisches Patentamt
European Patent Office
Office européen des brevets

(11) Publication number: **0 319 471 A2**

(12) **EUROPEAN PATENT APPLICATION**

(21) Application number: **88810797.6**

(22) Date of filing: **21.11.88**

(51) Int. Cl.4: **C 12 P 41/00**
C 12 P 13/00
// C12N9/84

(30) Priority: **01.12.87 GB 8728121**

(43) Date of publication of application:
**07.06.89 Bulletin 89/23**

(84) Designated Contracting States:
**BE CH DE FR GB GR IT LI NL**

(71) Applicant: **CIBA-GEIGY AG**
**Klybeckstrasse 141**
**CH-4002 Basel (CH)**

(72) Inventor: **Dingwall, John Grey, Dr.**
**St. Pantaleonstrasse 16**
**CH-4412 Nuglar (CH)**

**Baylis, Eric Keith, Dr.**
**18, Abbey Grove**
**Offerton Stockport Cheshire (GB)**

**Brundish, Derek Edward, Dr.**
**70 Smithbarn**
**Horsham West Sussex RH13 6DU (GB)**

(54) **Resolution process.**

(57) The invention provides a process for the production of the enantiomers of an aminophosphonous acid having the formula (I), or the corresponding zwitterion form:

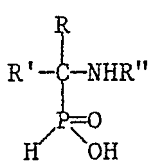

I

in which
R and R′ are different and each is hydrogen; $C_1$-$C_6$alkyl; $C_1$-$C_6$alkyl substituted by i) one or two $-COOR^2$, $-OR^2$, $-SR^2$ or $-CONH_2$ groups, in which $R^2$ is hydrogen, phenyl, or $C_1$-$C_3$alkyl which is optionally substituted by a $C_6$-$C_{10}$aryl radical; ii) by an $-SS-CH_2-CH(NH_2)-PO_2H_2$ group; iii) by $-NH_2$ or protected amino; iv) by an $-NH-C(=NH)NH_2$ group; v) by a $C_6$-$C_{10}$aryl radical which is optionally substituted by one or two hydroxyl groups; or vi) by a heterocyclic ring having 3 to 7 ring atoms including one or more nitrogen atoms, which ring is optionally fused to a benzene ring; and R″ is hydrogen or $C_1$-$C_6$alkyl or R″, together with the -NH-C(R)(R′)- residue to which it is bonded, are the atoms required to complete a pyrrolidin-2-yl or piperidin-2-yl ring which is optionally substituted by a hydroxyl group or by a group $R^2$; which process comprises:

a) selectively cleaving, using Penicillin-G-amidase, one enantiomer of a racemic mixture of N-acyl derivatives of the aminophosphonous acid of formula I, the N-acyl derivative having the formula II:

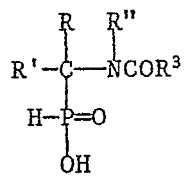

II

wherein R, R′ and R″ are as defined under formula I, and $R^3$ is a straight- or branched $C_1$-$C_6$alkyl group which is optionally substituted by one or more of halogen, hydroxy, $C_1$-$C_3$alkoxy, phenyl, phenoxy and/or thienyl, the phenyl- or phenoxy substituents being themselves optionally substituted by $C_1$-$C_3$alkyl, hydroxy, nitro, amino, halogen and/or $C_1$-$C_3$alkoxy;

b) separating the mixture of free amino acid and unhydrolysed N-acyl derivative of formula II; and

c) de-acylating, non-enzymatically, the un-hydrolysed material.

EP 0 319 471 A2

## Description

### Resolution Process

The present invention relates to a resolution process, in particular to a process for producing the enantiomers of amino phosphonous acids.

Certain amino phosphonous acids have been shown, in European Patent 2031, to have useful plant growth regulatory properties and, in British Patent Specification No. 1542938, to exhibit interesting antimicrobial activity. Moreover, in European Patent 10066, there are described peptide derivatives of amino phosphonous acids having plant growth regulating properties.

It is well established that one particular enantiomer of a racemic mixture can exhibit a much higher activity relative to other enantiomers, or to the total racemic mixture.

There is an interest, therefore, in devising a process for effectively and efficiently resolving racemic mixtures of aminophosphonous acids, as produced e.g. in the synthetic methods described in GB 1542938, in order to separate individual enantiomers from the racemic mixtures.

In European Patent Specification No. 0176068, there is described a process for the production of the enantiomers of 1-aminoalkylphosphonic acids or 1-aminoalkylphosphinic acids by selective enzymatic hydrolysis of one enantiomer of their racemic N-acyl derivatives; and subsequently de-acylation of the unhydrolysed N-acyl derivative. The process is characterised in that the enzymatic resolution is performed using Penicillin-G-amidase.

There is no suggestion in this specification that the process claimed therein could be used to resolve racemic mixtures of starting materials other than the N-acyl derivatives of the said phosphonic or - phosphinic acids.

Moreover, it is stated in the paper "1-Aminoalkylphosphonous Acids", by Baylis et al., J. Chem. Soc. Perkin Trans. I. 1984, pages 2845 to 2853, at page 2848, that "preliminary mode of action studies using enzymes with (-)-$(CH_3)_2CHCH(NH_2)PH(=O)OH$ showed that it was antagonised by L-valine and inhibited the protein synthesis of E. Coli B...". Moreover, the valyl and methionyl phosphonous analogues are stated, in FEBS Letters, 1978, 9, 246 to be inhibitors for transfer-RNA synthetases.

In view of this prejudice against the use of amino phosphonous acids in combination with enzymes, surprisingly we have now found that certain racemic mixtures of amino phosphonous acids, or their derivatives, can be resolved into the respective enantiomers, effectively and efficiently, using a specific enzyme, namely Penicillin-G-amidase.

Accordingly, the present invention provides a process for the production of the enantiomers of an aminophosphonous acid having the formula (I), or the corresponding zwitterion form

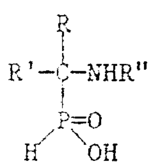

I

in which
R and R′ are different and each is hydrogen; $C_1$-$C_6$alkyl; $C_1$-$C_6$alkyl substituted by i) one or two $-COOR^2$, $-OR^2$, $-SR^2$ or $-CONH_2$ groups, in which $R^2$ is hydrogen, phenyl, or $C_1$-$C_3$alkyl which is optionally substituted by a $C_6$-$C_{10}$aryl radical; ii) by an $-SS-CH_2-CH(NH_2)-PO_2H_2$ group; iii) by $-NH_2$ or protected amino; iv) by an $-NH-C(=NH)NH_2$ group; v) by a $C_6$-$C_{10}$aryl radical which is optionally substituted by one or two hydroxyl groups; or vi) by a heterocyclic ring having 3 to 7 ring atoms including one or more nitrogen atoms, which ring is optionally fused to a benzene ring; and R″ is hydrogen or $C_1$-$C_6$alkyl, preferably methyl more especially hydrogen, or R″, together with the -NH-C(R)(R′)- residue to which it is bonded, are the atoms required to complete a pyrrolidin-2-yl or piperidin-2-yl ring which is optionally substituted by a hydroxyl group or by a group $R^2$ wherein $R^2$ has its previous significance; which process comprises:

a) selectively cleaving, using Penicillin-G-amidase, one enantiomer of a racemic mixture of N-acyl derivatives of the aminophosphonous acid of formula I, the N-acyl derivative having the formula II:

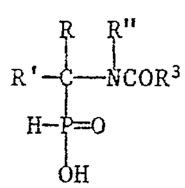

II

wherein R, R′ and R″, have their previous significance and $R^3$ is a straight- or branched $C_1$-$C_6$alkyl group which is optionally substituted by one or more of halogen, hydroxy, $C_1$-$C_3$alkoxy, phenyl, phenoxy and/or thienyl, the phenyl- or phenoxy substituents being themselves optionally substituted by $C_1$-$C_3$alkyl, hydroxy, nitro, amino, halogen and/or $C_1$-$C_3$alkoxy;

b) separating the mixture of free amino acid and unhydrolysed N-acyl derivative of formula II; and

c) de-acylating, non-enzymatically, the un-hydrolysed material.

$C_1$-$C_6$Alkyl groups R, R′, R″ or $R^3$ include methyl, ethyl, n-propyl, isopropyl, n-butyl, sec-butyl, tert-butyl, n-pentyl and n-hexyl.

The compounds of formula II are further object of this invention.

Any protecting group, e.g. an acyl or oxycarbonyl group, on an amino group substituent of R or R′ or an ester group on an alkyl group R or R′ may be removed by conventional methods after step b), preferably simultaneously with step c).

When R or R′ is $C_1$-$C_6$alkyl substituted by one or two $-COOR^2$, $OR^2$ or $SR^2$ groups, the $R^2$ substituent may be e.g. hydrogen, phenyl, methyl, ethyl or benzyl.

When R or R′ is $C_1$-$C_6$alkyl substituted by an optionally protected $-NH_2$ group, this aminoalkyl group may contain an amino group in a protected amide form, such as benzoyl or acetyl form, or in a protected urethane form e.g. as the benzyloxycarbonyl form. These amine protected forms may be produced by methods which are well-known to the art-skilled.

When R or R′ is $C_1$-$C_6$alkyl substituted by an optionally hydroxy-substituted $C_6$-$C_{10}$aryl group, this aryl group may be phenyl, 4-hydroxyphenyl or 3,4-dihydroxyphenyl.

R or R′ as $C_1$-$C_6$alkyl substituted by a heterocyclic ring may contain an imidazole or indole ring as such heterocyclic substituents.

When $R^3$ is $C_1$-$C_6$alkyl substituted by halogen, such halogen substituents may be, e.g. fluorine, chlorine or bromine substituents.

$R^3$ as $C_1$-$C_6$alkyl substituted by $C_1$-$C_3$alkoxy may contain methoxy, ethoxy or n-propoxy as such alkoxy substituents.

$R^3$ as $C_1$-$C_6$alkyl substituted by optionally substituted phenyl or phenoxy groups may contain phenyl, phenoxy, p-tolyl, 4-hydroxyphenyl, 4-nitrophenyl, 4-aminophenyl, 4-chlorophenyl or 4-methoxyphenyl as such phenyl or phenoxy substituents.

$R^3$ as $C_1$-$C_6$alkyl substituted by both halogen, e.g. fluorine, chlorine or bromine, and by optionally substituted phenyl, e.g. p-tolyl, may be, e.g., α-fluorobenzyl or α-fluoro-α-phenyl ethyl.

Preferably, $R^3$ is unsubstituted $C_1$-$C_6$alkyl; or $C_1$-$C_6$alkyl, preferably methyl, substituted by phenyl.

In a preferred embodiment R″ is hydrogen and $R^3$ is unsubstituted $C_1$-$C_6$alkyl; or $C_1$-$C_6$alkyl, halo-$C_1$-$C_6$alkyl or $C_1$-$C_3$alkoxy-$C_1$-$C_6$alkoxy-each substituted by phenyl.

In another preferred embodiment R″ is hydrogen and $R^3$ is unsubstituted $C_1$-$C_6$alkyl, benzyl or α-fluorbenzyl.

In still another embodiment, R′ and R″ are hydrogen and R is benzyl,

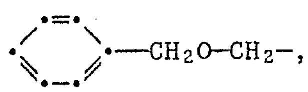, $HOOC(CH_2)_2-$, $H_2N(CH_2)_3-$ or

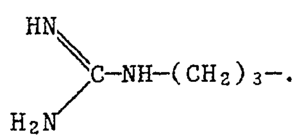

Specific examples of aminophosphonous acids of formula I which may be resolved by the process of the present invention include those having the formula:

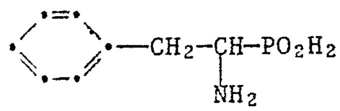

III

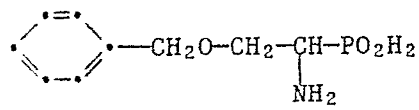

IV

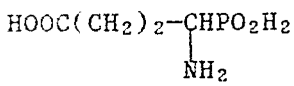

V

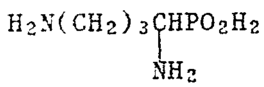

VI

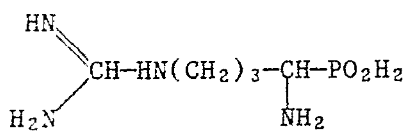

VII

The new N-acyl derivatives of acids of formula II which form a further aspect of the present invention, can be produced by methods conventional in organic chemistry, by reacting the corresponding amino phosphonous acid with activated carboxylic acid derivatives, or carboxylic acids in the presence of a condensation agent. Activated carboxylic acid derivatives are, e.g. acid chlorides, symmetrical anhydrides or mixed anhydrides with carbonic acid alkyl esters, active esters e.g. p-nitrophenyl esters, N-hydroxysuccinimide or 1-N-hydroxy-benzotriazole esters. Condensation agents are e.g. carbodiimides.

Alternatively, the amino phosphonous acids can be N-acylated in the form of their alkyl esters or trialkylsilyl esters, using an activated carboxylic acid. After the acylation reaction, the phosphonous acid alkyl ester can be cleaved e.g. by reaction with a base or with HBr in glacial acetic acid. Trialkylsilyl esters are very readily hydrolysed using water.

Depending on the hydrolytic stability of the reactants, the acylation reaction can be performed in water or aqueous/alcohol mixtures, or in inert organic solvents e.g. methylene chloride or acetone.

Racemic amino phosphonous acids of formula I are known compounds having been described e.g. in British Patent Specification No. 1542938 and in European Patent Specification No. 2039.

The Penicillin-G-amidase used according to the invention can be employed as the free, water-soluble enzyme e.g. as the lyophilisate or, preferably, as the immobilised enzyme in water-insoluble form. It is preferably formed from Escherichia coli (M. Cole et al. Meth. Enzym. 43 (1975) 698) which is known under the E.C. Number 3.5.1.11, e.g. Penicillin-G-amidase from E. Coli DSM 1900 (ATCC 11105).

For larger charges on technical scale, it is preferable to use the enzyme in immobilised form. The reaction can then be performed in a reactor, e.g. a column reactor, suitable for immobilised enzyme.

Water is the preferred reaction medium. Water-miscible solvents e.g. alcohols such as ethanol may also be present in addition to the water.

The substrate concentration lies in general within the range of form 0.1 molar up to the solubility limit in aqueous or aqueous-organic media.

The preferred reaction temperature ranges from 20 to 60°C. Most preferred being the range 35-40°C.

The process of the invention is conveniently conducted at a pH value ranging from 5 to 8 and optionally in the presence of a buffer e.g. a phosphate buffer. The preferred pH is about 7.

The enzymatic cleavage can be readily monitored by NMR analytical methods. The stereo specific cleavage can also be monitored by a polarimetric measurement of the isolated R- and S-aminophosphonous acid. Alternatively and advantageously, use can be made of a R or S chiral amide derivative e.g. a compound having the formula IIa or IIb

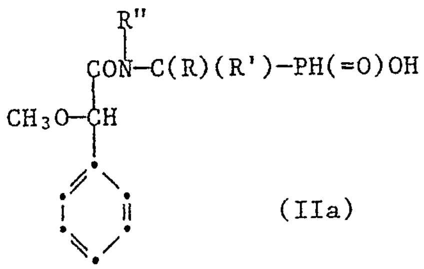
(IIa)

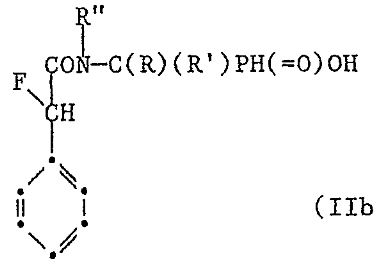
(IIb)

to monitor the cleavage by NMR methods, without the prior isolation of the R- und S-aminophosphonous acids.

The reaction mixtures of the enzymatic cleavage may be worked up by chromatographic methods on a strongly acidic ion exchanger in the $H^+$ form. Using water as elution means, there are eluted, consecutively the carboxylic acid of the acyl residue, the unhydrolysed enantiomer of the acylaminophosphonous acid, and then the amino phosphonous acid in pure form. The acylamino phosphonous acid can be conveniently de-acylated by boiling with aqueous hydrochloric acid.

The R- and S-isomers of the amino phosphonous acid so processed, are obtained in an optical purity above 95%.

The enantiomers of amino phosphonous acids, produced according to the process of the present invention, are useful in a wide range of applications. As mentioned hereinbefore, they have interesting antimicrobial and plant growth regulatory properties. Moreover, they are useful as intermediates for the corresponding amino phosphonic and -phosphinic acids.

The following Examples further illustrate the present invention. Examples A to E relate to the preparation of novel starting materials of formula II. Examples 1 to 5 relate to the production of compounds of formula I by the process of the invention.

Examples A to E: Synthesis of 1-acylaminophosphonous acids

A) 1-amino-2-phenylethylphosphonous acid (1.85 g, 0.01M) is stirred in deionised water (40 ml) at room temperature. Sodium hydroxide (2M) is added dropwise until pH 10 and the solution has become clear. Phenyl acetyl chloride (1.70 g, 0.011M) is added, dropwise, at room temperature and sodium hydroxide is added, dropwise, to maintain the pH at 10. The reaction mixture is stirred at room temperature for 1 hour, cooled to 0° and acidified to pH 1 with c.HCl. A white solid forms which is filtered off and dried to give 1-phenylacetamido-2-phenylethyl phosphonous acid.

Similarly prepared are the 1-acylamino phosphonous acids of formula

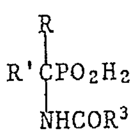

and shown in Table 1.

Table 1

| Example | R | R' | $R^3$ | m.p.° | $^{31}P$ | Max. IR $cm^{-1}$ | Yield % |
|---|---|---|---|---|---|---|---|
| B | H | $C_6H_5CH_2-$ | $CH_3CH_2CH_2-$ | 121 | 26.9 | 1640<br>3240 | 61 |
| C | H | 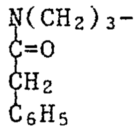 | $C_6H_5CH_2-$ | 156 | 27.9 | 1650<br>3240 | 26 |
| D | H | 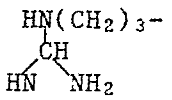 | $C_6H_5CH_2-$ | 258 | 24.5 | 1640<br>3200 | 38 |
| E | H | $C_6H_5CH_2OCH_2-$ | $C_6H_5CH_2-$ | 150 | 24.4 | 1650<br>3240 | 65 |
| F | H | $C_6H_5CH_2-$ | $C_6H_5CHF-$ | – | – | – | – |

Example 1:

i) R,S-1-Phenylacetamido-2-benzyloxyethanephosphonous acid prepared according to Example E (4.3 g., 0.13M), water (280 ml), sodium bicarbonate (4.0 g) and Penicillin-G-amidase (Rohm Pharma 1.72 g suspension) are stirred at 37° under nitrogen for 48 hours. The enzyme is filtered off and the filtrate is evaporated to a gummy solid. This solid is dissolved in water and the mixture chromatographed on ion-exchange resin, Dowex 50WX2. It gives, firstly, phenylacetic acid, then S-1-phenylacetamido-2-benzyoxyethane phosphonous acid (2 g) and finally R-1-amino-2-benzyloxyethanephosphonous acid (0.6 g).

ii) The R-1-amino-2-benzyloxyethanephosphonous acid (0.6 g) and c·HCl (30 ml) are refluxed for 8 hours. The mixture is evaporated to dryness and the residue taken up in water and washed with ether. The aqueous liquors are evaporated to dryness and the residue is dissolved in ethanol. Propylene oxide is added, dropwise, and the mixture stirred for 24 hours. The solid which separates is filtered off to give R-1-amino-2-hydroxyethane phosphonous acid (0.28 g., m.p. 220°).

The optical purity is determined by $^{31}P$ and $^{19}F$ NMR analysis of the amide of Mosher's acid prepared from the aminophosphonous acid and R(-) or S(-)-α-methoxy-α-trifluoromethylphenylacetyl chloride (Dale, Dull and Mosher, J. Org. Chem. 1969, 34, 2543). Thus, this solid (0.0123 g, 0.1 mm) is suspended in water (0.7 ml) and sodium bicarbonate (0.0336 g, 40 mm) is added. The mixture is stirred and gives a pH 9.5. p-Dioxane 1.4 ml is added followed by R(+)-α-methoxy-α-trifluoromethylphenylacetyl chloride (26 µl) and the mixture stirred for 30 minutes. The mixture is evaporated to one third volume, water is added to give a clear solution and the NMR spectra are run. The $^{31}P$ NMR spectrum shows one signal at 20.18 ppm and the $^{19}F$ spectrum only one signal at -66.76 ppm, other than that due to the excess of Mosher reagent, indicating optical purity. The absolute configuration is determined by mercuric chloride oxidation (E.K.Baylis et al., J. Chem. Soc. Perkin Trans I, 1984, 2845) to R-1-amino-2-hydroxyethanephosphonic acid, $[\alpha]_D^{20}$ -27° (c.f. Mastalerz et al., Phosphorus and Sulfur 1983, Vol. 18, p. 393).

iii) The S-1-phenylacetamido-2-benzyloxyethane phosphonous acid (0.9 g) and c·HCl (15 ml) are refluxed for 8 hours. The mixture is evaporated to dryness, the residue taken up in water, washed with ether and again evaporated to dryness. The residue is dissolved in a little alcohol and propylene oxide added dropwise. The solid which forms is filtered off to give S-1-amino-2-hydroxyethanephosphonous acid (0.22 g, m.p. 220°. $^{31}P$ NMR one signal 19.80 ppm and $^{19}F$ NMR one signal -66.78 ppm other than that of excess of Mosher reagent.)

Example 2:

A solution of R,S-1-Phenylacetamido-2-phenyl ethanephosphonous acid (0.75 g) prepared according to Example A, sodium bicarbonate (0.7 g) and water (40 ml) is stirred with Penicillin-G-amidase (0.3 g

suspension) for 72 hours at 37°. The enzyme is filtered off and the solution evaporated to dryness. The resulting solid is chromatographed on Dowex 50 w x 2 to give S-1-phenylacetamido-2-phenylethanephosphonous acid and R-1-amino-2-phenylethane phosphonous acid.

$^{31}P$ NMR of the R(+)- Mosher derivative derived from R-1-amino-2-phenylethane phosphonous acid shows one signal 21.43 pm and $^{19}F$ NMR one signal -67.14 ppm. Hydrolysis of the phenylacetamido as in Example 1 gives S-1-amino-2-phenylethanephosphonous acid. $^{31}P$ NMR of the R(+)-Mosher derivative shows one signal 21.06 ppm and $^{19}F$ NMR shows one signal -67.27 ppm. The absolute configuration is confirmed by peak enhancement using the Mosher derivatives of R- and S-1-amino-2-phenylethanephosphonous acids obtained by classical resolution (E.K. Baylis loc. cit.).

Example 3:

Using the procedure described in Example 2, on treatment with Penicillin-G-amidase, R,S-1-butyramido-2-phenylethanephosphonous acid prepared according to Example B gives S-1-butyramido-2-phenylethanephosphonous acid and R-1-amino-2-phenylethanephosphonous acid.

$^{31}p$ NMR of the R(+)-Mosher derivative of the latter shows one signal 21.43 ppm and $^{19}F$ NMR one signal -67.14 ppm.

Example 4:

Using the procedure described in Example 2, on treatment with Penicillin-G-amidase, R,S- 1,4-bis-phenylacetamidobutane phosphonous acid prepared according to Example C gives S-1,4-bis-phenylacetamidobutane phosphonous acid and R-1,4-diaminobutane phosphonous acid.

$^{31}P$ NMR of the S(-)-Mosher derivative of the latter shows one signal 24.7 ppm and $^{19}F$ NMR one signal -66.7 ppm.

Example 5:

Using the method described in Example 2, on treatment with Penicillin-G-amidase R,S-1-phenylacetamido-4-guanidinobutane phosphonous acid prepared according to Example D gives S-1-phenylacetamido-4-guanidinobutane phosphonous acid and R-1-amino-4-guanidinobutane phosphonous acid.

$^{31}P$ NMR of the S(-)-Mosher derivative of the latter shows one signal 21.98 ppm and $^{19}F$ NMR one signal -68.92 ppm.

In a manner analogous to that described in Example 1, the S-1-phenylacetamido-4-guanidinobutane phosphonous acid is treated with concentrated HCl, to give S-1-amino-4-guanidinobutane phosphonous acid. The $^{31}P$ NMR of the S(-)-Mosher derivative of the latter shows one signal at 22.58 ppm, and the $^{19}F$ NMR shows one signal at -66.899 ppm.

Example 6:

α-Fluorophenylacetic acid is resolved using (-)-Ephedrine to give (-)-α-fluorophenylacetic acid ($[\alpha]_D^{25}$ -141.6°C 1.5 % acetone) (J. Chem. Soc. Perkin II 1977, 677). Part of which is converted to its acid chloride (b.p. 0.1 mbar/25°) by refluxing for 85 hours with 10 parts by weight of thionyl chloride.

Using the procedure described in Example 2, on treatment with penicillin amidase, R,S-1-(-)-α-fluorophenylacetamido-2-phenylethanephosphonous acid, prepared according to Example F, gives S-1-(-)-α-fluorophenylacetamido-2-phenylethanephosphonous acid and R-1-amino-2-phenylethanephosphonous acid. By using this chiral derivative, the hydrolysis is monitored using $^{19}F$ and $^{31}P$ NMR analysis. Thus, the $^{19}F$ NMR spectrum of the starting amide derivative in sodium bicarbonate shows two doublets due to the R-(-)- and S-(-)-enantiomers (F-H coupled) at -168.01, -168.56 and -170.58, -171.13 ppm respectively. Likewise, the $^{31}P$ NMR spectrum shows the two corresponding P signals at 23.56 and 24.21 ppm. As the hydrolysis proceeds, the peaks due to the R-(-)-enantiomer disappear and these are replaced by a doublet due to (-)-α-fluoroacetic acid (H-F coupled) at -160.93 and -161.54 ppm in the $^{19}F$ NMR and a single P-signal due to R-1-amino-2-phenylethanephosphonous acid at 30.85 ppm in the $^{31}P$ NMR spectra.

Note: All $^{31}P$ NMR spectra are referred to 80 % $H_3PO_4$ and $^{19}F$ NMR spectra to $CFCl_3$.

## Claims

1. Process for the production of the enantiomers of an aminophosphonous acid having the formula (I), or the corresponding zwitterion form:

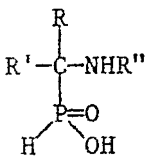

I

in which
R and R′ are different and each is hydrogen; $C_1$-$C_6$alkyl; $C_1$-$C_6$alkyl substituted by i) one or two -$COOR^2$, -$OR^2$, -$SR^2$ or -$CONH_2$ groups, in which $R^2$ is hydrogen, phenyl, or $C_1$-$C_3$alkyl which is optionally substituted by a $C_6$-$C_{10}$aryl radical; ii) by an -SS-$CH_2$-CH($NH_2$)-$PO_2H_2$ group; iii) by -$NH_2$ or protected amino; iv) by an -NH-C(=NH)$NH_2$ group; v) by a $C_6$-$C_{10}$aryl radical which is optionally substituted by one or two hydroxyl groups; or vi) by a heterocyclic ring having 3 to 7 ring atoms including one or more nitrogen atoms, which ring is optionally fused to a benzene ring; and R″ is hydrogen or $C_1$-$C_6$alkyl or R″, together with the -NH-C(R)(R′)- residue to which it is bonded, are the atoms required to complete a pyrrolidin-2-yl or piperidin-2-yl ring which is optionally substituted by a hydroxyl group or by a group $R^2$ wherein $R^2$ has its previous significance; which process comprises:

a) selectively cleaving, using Penicillin-G-amidase, one enantiomer of a racemic mixture of N-acyl derivatives of the aminophosphonous acid of formula I, the N-acyl derivative having the formula II:

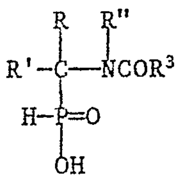

II

wherein R, R′ and R″, are as defined under formula I, and $R^3$ is a straight- or branched $C_1$-$C_6$alkyl group which is optionally substituted by one or more of halogen, hydroxy, $C_1$-$C_3$alkoxy, phenyl, phenoxy and/or thienyl, the phenyl- or phenoxy substituents being themselves optionally substituted by $C_1$-$C_3$alkyl, hydroxy, nitro, amino, halogen and/or $C_1$-$C_3$alkoxy;

b) separating the mixture of free amino acid and unhydrolysed N-acyl derivative of formula II; and

c) de-acylating, non-enzymatically, the un-hydrolysed material.

2. Process according to claim 1 wherein the aminophosphonous acid racemate of formula I has the formula III, IV, V, VI or VII:

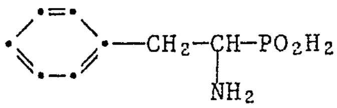

III

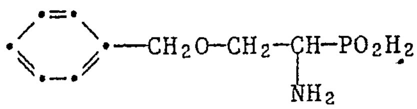

IV

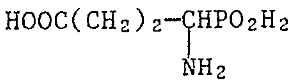

V

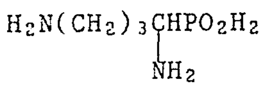

VI

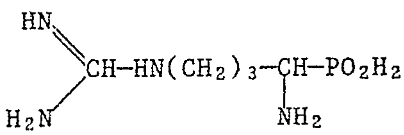

VII.

3. Process according to claim 1 wherein the Penicillin-G-amidase used is formed from Escherichia coli (E. Coli) which is known under the E.C. Number 3.5.1.11.

4. Process according to claim 3 wherein the Penicillin-G-amidase is used as the immobilised enzyme in water-insoluble form.

5. Process according to claim 1 wherein water, optionally containing water-miscible solvents, is used as the reaction medium.

6. Process according to claim 1 wherein the reaction temperature ranges from 20 to 60° C.

7. Process according to claim 1 wherein the pH ranges from 5 to 8.

8. N-acyl derivatives having the formula II:

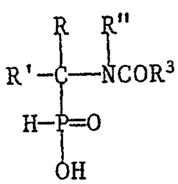

II

wherein R, R′ and R″ are as defined in claim 1 and $R^3$ is a straight- or branched $C_1$-$C_6$alkyl group which is optionally substituted by halogen, hydroxy, $C_1$-$C_3$alkoxy, phenyl, phenoxy and/or thienyl, the phenyl- or phenoxy substituents being themselves optionally substituted by $C_1$-$C_3$alkyl, hydroxy, nitro, amino, halogen and/or $C_1$-$C_3$alkoxy.

9. N-acyl derivatives of formula II according to claim 8 wherein R″ is hydrogen and $R^3$ is unsubstituted $C_1$-$C_6$alkyl; or $C_1$-$C_6$alkyl, halo-$C_1$-$C_6$alkyl or $C_1$-$C_3$alkoxy-$C_1$-$C_6$alkoxy- each substituted by phenyl.

10. N-acyl derivatives according to claim 9 wherein R″ is hydrogen and $R^3$ is unsubstituted $C_1$-$C_6$alkyl, benzyl or α-fluorbenzyl.

11. N-acyl derivatives according to claim 8 wherein R′ and R″ are hydrogen and R is benzyl,

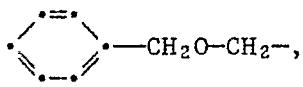, $HOOC(CH_2)_2-$, $H_2N(CH_2)_3-$ or